(19) Europäisches Patentamt
European Patent Office
Office européen des brevets

(11) EP 2 964 812 B1

(12) **EUROPÄISCHE PATENTSCHRIFT**

(45) Veröffentlichungstag und Bekanntmachung des Hinweises auf die Patenterteilung:
**21.03.2018 Patentblatt 2018/12**

(21) Anmeldenummer: **13811148.9**

(22) Anmeldetag: **10.12.2013**

(51) Int Cl.:
***C25B 3/10*** *(2006.01)* ***C07C 43/23*** *(2006.01)*

(86) Internationale Anmeldenummer:
**PCT/EP2013/076086**

(87) Internationale Veröffentlichungsnummer:
**WO 2014/135237 (12.09.2014 Gazette 2014/37)**

(54) **ELEKTROCHEMISCHE KUPPLUNG EINES PHENOLS MIT EINEM NAPHTHOL**
ELECTROCHEMICAL COUPLING OF A PHENOL TO A NAPHTHOL
COUPLAGE ÉLECTROCHIMIQUE D'UN PHÉNOL À UN NAPHTHOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.03.2013 DE 102013203866**

(43) Veröffentlichungstag der Anmeldung:
**13.01.2016 Patentblatt 2016/02**

(73) Patentinhaber: **Evonik Degussa GmbH**
**45128 Essen (DE)**

(72) Erfinder:
- **DYBALLA, Katrin Marie**
  **45657 Recklinghausen (DE)**
- **FRANKE, Robert**
  **45772 Marl (DE)**
- **FRIDAG, Dirk**
  **45721 Haltern am See (DE)**
- **WALDVOGEL, Siegfried R.**
  **55435 Gau-Algesheim (DE)**
- **ELSLER, Bernd**
  **53127 Bonn (DE)**

(56) Entgegenhaltungen:
**WO-A1-2010/139685**

- **SARTORI ET AL.: "Selective Synthesis of unsymmetrical Selective synthesis of unsymmetrical 2,2'-dihydroxylated biaryls via electrophilic arylation of metal phenolates with p-benzoquinone monoketals", JOURNAL OF THE CHEMICAL SOCIETY, PERKIN TRANSACTIONS 1, Nr. 17, 1995, Seiten 2177-2182, XP002722558,**
- **KUMAR ET AL.: "Reversal of Polarity in Masked o-Benzoquinones: Rapid Access to Unsymmetrical Oxygenated Biaryls", ORGANIC LETTERS, Bd. 15, Nr. 14, 2. Juli 2013 (2013-07-02), Seiten 3546-3549, XP002722559,**
- **KIRSTE A ET AL: "Anodic Phenol-Arene Cross-Coupling Reaction on Boron-Doped Diamond Electrodes", ANGEWANDTE CHEMIE. INTERNATIONAL EDITION, VCH VERLAG, WEINHEIM, DE, Bd. 49, Nr. 5, 25. Januar 2010 (2010-01-25), Seiten 971-975, XP002595230, ISSN: 0570-0833, DOI: 10.1002/ANIE.200904763 [gefunden am 2009-12-22]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die folgende Erfindung betrifft ein elektrochemisches Verfahren zur Kupplung von einem Phenol mit einem Naphthol, welche sich in ihrem Oxidationspotential unterscheiden.

Die Begriffe Phenole und Naphthole werden in dieser Anmeldung als Gattungsbegriff verwendet und umfassen somit auch substituierte Phenole bzw. substituierte Naphthole.

Eine elektrochemische Kupplung wurde bis jetzt nur von Phenol mit einem Naphthol beschrieben, welches keine OH-Gruppe aufweist: Kirste et al. Angew. Chem. 2010, 122, 983-987 und Kirste et al. J. Am. Chem. Soc. 2012, 134, 3571-3576.

In KIRSTE A ET AL, "Anodic Phenol-Arene Cross-Coupling Reaction on Boron-Doped Diamond Electrodes", ANGEWANDTE CHEMIE. INTERNATIONAL EDITION, VCH VERLAG, WEINHEIM, DE, Vol. 49, Nr. 5, Seiten 971 - 975, wird eine anodische Phenol-Aren Kreuzkupplung beschrieben. Die Kupplung erfolgt hierbei an einer BDD-Anode (BDD = boron-doped diamond).

In der WO 2010/139685 A1 wird ein Verfahren zur anodischen Kreuz-Dehydrodimerisierung von Arenen beschreiben. Herbei wird in Gegenwart von teil- und/oder perfluorierten Mediatoren und einem Leitsalz gearbeitet.

Ein Problem, das bei der elektrochemischen Kupplung von unterschiedlichen Molekülen auftritt ist, dass diese unterschiedliche Oxidationspotentiale $E_{Ox}$ haben. Dies hat zur Folge, dass das Molekül mit dem höheren Oxidationspotential ein geringeres Bestreben hat, ein Elektron ($e^-$) an die Anode und ein $H^+$-Ion an z.B. das Lösungsmittel abzugeben, als das Molekül mit dem niedrigeren Oxidationspotential. Berechnen lässt sich das Oxidationspotential $E_{Ox}$ über die Nernst-Gleichung:

$$E_{Ox} = E° + (0{,}059/n) * \lg([Ox]/[Red])$$

$E_{Ox}$: Elektrodenpotential für die Oxidationsreaktion (= Oxidationspotential)
$E°$: Standardelektrodenpotential
*n*: Anzahl der übertragenen Elektronen
*[Ox]*: Konzentration der oxidierten Form
*[Red]:* Konzentration der reduzierten Form

Die Aufgabe der folgenden Erfindung bestand darin, ein elektrochemisches Verfahren bereitzustellen, bei dem ein Phenol mit einem Naphthol gekuppelt wird, wobei diese beiden Moleküle unterschiedliche Oxidationspotentiale aufweisen.

Elektrochemisches Verfahren umfassend die Verfahrensschritte:

a) Einfüllen eines Lösungsmittels oder Lösungsmittelgemisches sowie eines Leitsalz, in ein Reaktionsgefäß,
b) Zugabe eines Phenols mit einem Oxidationspotential $|E_{Ox}1|$ in das Reaktionsgefäß,
c) Zugabe eines Naphthols mit einem Oxidationspotential $|E_{Ox}2|$ in das Reaktionsgefäß, wobei die Substanz mit dem höheren Oxidationspotential im Überschuss zugesetzt wird,
und es gilt: $|E_{Ox}1| - |E_{Ox}2| = |\Delta E|$
und das Lösungsmittel oder Lösungsmittelgemisch so gewählt ist, dass $|\Delta E|$ im Bereich von 10 bis 450 mV liegt,
d) Einbringen zweier Elektroden in die Reaktionslösung,
e) Anlegen einer Spannung an die Elektroden,
f) Kupplung des Phenols mit dem Naphthol zu einem kreuzgekuppeltem Produkt.

Die Verfahrensschritte a) bis d) können hierbei in beliebiger Reihenfolge erfolgen.

Das Verfahren kann an unterschiedlichen Kohlenstoff- (Glaskohlenstoff, Bor-dotierter Diamant, Graphiten, Kohlenstoffasern, Nanotubes, u.a.), Metalloxid- und Metallelektroden durchgeführt werden. Dabei werden Stromdichten im Bereich von 1-50 $mA/cm^2$ appliziert.

Mit dem erfindungsgemäßen Verfahren wird das zu Beginn genannte Problem gelöst.

Ein Teilaspekt der Erfindung besteht darin, dass sich die Ausbeute der Reaktion über die Differenz der Oxidationspotentiale ($|\Delta E|$) der beiden Verbindungen steuern lässt.

Für eine effiziente Reaktionsführung sind zwei Reaktionsbedingungen notwendig:

- die Verbindung mit dem höheren Oxidationspotential muss im Überschuss zugegeben werden, und
- die Differenz der beiden Oxidationspotentiale ($|\Delta E|$), muss in einem bestimmten Bereich liegen.

Ist die erste Bedingung nicht erfüllt, so entsteht als Hauptprodukt eine Verbindung, welche durch die Kupplung

aus zwei Molekülen mit dem niedrigeren Oxidationspotential entsteht.

Ist |*ΔE*| zu klein so entsteht zu viel Nebenprodukt der Verbindung, welche durch die Kupplung aus zwei Molekülen mit dem höheren Oxidationspotential entsteht, da dieses im Überschuss zugesetzt wird.

Ist dagegen |*ΔE*| zu groß, so würde ein zu hoher Überschuss an der Verbindung mit dem höheren Oxidationspotential benötigt, was die Reaktion unrentabel machen würde.

Für das erfindungsgemäße Verfahren ist die Kenntnis der absoluten Oxidationspotentiale der beiden Verbindungen nicht zwingend notwendig. Es ist ausreichend, wenn die Differenz der beiden Oxidationspotentiale zueinander bekannt ist.

Ein weiterer Teilaspekt der Erfindung ist, dass sich die Differenz der beiden Oxidationspotentiale (|*ΔE*|), über die verwendeten Lösungsmittel bzw. Lösungsmittelgemische beeinflussen lässt.

So kann die Differenz der beiden Oxidationspotentiale (|*ΔE*|) durch geeignete Wahl des Lösungsmittels / Lösungsmittelgemisches in den gewünschten Bereich verschoben werden.

Geht man von 1,1,1,3,3,3-Hexafluorisopropanol (HFIP) als Basislösungsmittel aus, so lässt sich ein zu kleines |*ΔE*| beispielsweise durch Zugabe von Alkohol erhöhen. Ein zu großes |*ΔE*| kann hingegen durch Zugabe von Wasser abgesenkt werden.

Die ablaufende Reaktionsfolge ist in dem folgenden Schema dargestellt:

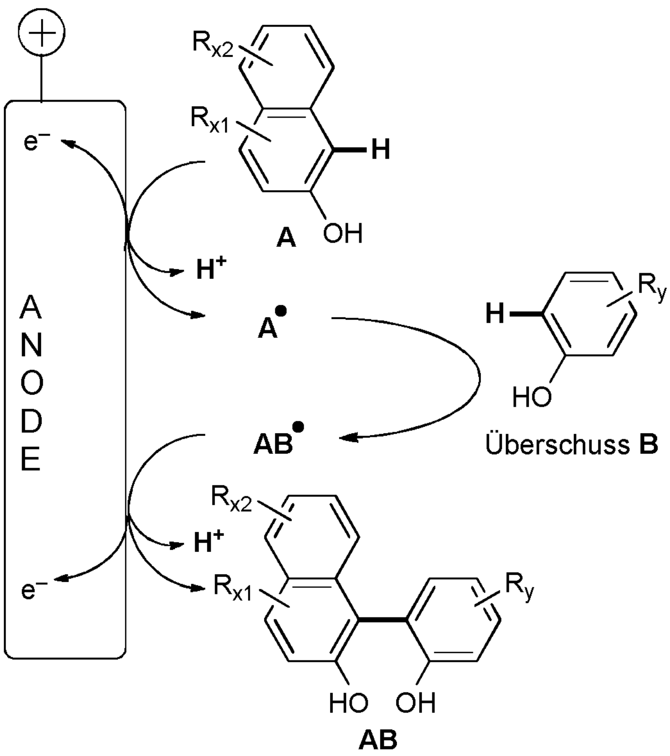


Zuerst gibt die Verbindung A, welche das niedrigere Oxidationspotential besitzt, ein Elektron an die Anode ab. Verbindung A wird aufgrund der positiven Ladung zu einer sehr starken Säure und spaltet spontan ein Proton ab. Das so entstandene Radikal reagiert anschließend mit der Verbindung B, welches gegenüber der Verbindung A im Überschuss in der Lösung vorhanden ist. Das durch die Kupplung entstandene AB-Radikal gibt ein Elektron an die Anode und ein Protons an das Lösungsmittel ab.

Wäre die Verbindung mit dem höheren Oxidationspotential nicht im Überschuss zugesetzt, so würde das A-Radikal mit einer zweiten Verbindung A zu der entsprechenden AA-Verbindung reagieren.

Mit Hilfe des erfindungsgemäßen Verfahrens konnten erstmals Phenole mit Naphtholen elektrochemisch in guten Ausbeuten gekuppelt werden.

In einer Variante des Verfahrens wird die Substanz mit dem höheren Oxidationspotential mindestens in der doppelten Menge gegenüber der Substanz mit dem niedrigeren Oxidationspotential eingesetzt.

In einer Variante des Verfahrens ist das Lösungsmittel oder Lösungsmittelgemisch so gewählt, dass |*ΔE*| im Bereich von 20 mV bis 400 mV liegt, vorzugsweise im Bereich von 30 mV bis 350 mV.

In einer Variante des Verfahrens ist das Leitsalz ausgewählt aus der Gruppe von Alkali-, Erdalkali-, Tetra($C_1$-$C_6$-alkyl)-ammonium-,1,3-Di($C_1$-$C_6$-alkyl)imidazolium oder Tetra($C_1$-$C_6$-alkyl)-phosphoniumsalzen.

In einer Variante des Verfahrens sind die Gegenionen der Leitsalze ausgewählt aus der Gruppe von Sulfat,

Hydrogensulfat, Alkylsulfate, Arylsulfate, Alkylsulfonate, Arylsulfonate, Halogenide, Phosphate, Carbonate, Alkylphosphate, Alkylcarbonate, Nitrat, Tetrafluorborat, Hexafluorphosphat, Hexafluorsilikat, Fluorid und Perchlorat.

In einer Variante des Verfahrens ist das Leitsalz ausgewählt aus Tetra-($C_1$-$C_6$-alkly)ammoniumsalzen und das Gegenion ausgewählt aus Sulfat, Alkylsulfat, Arylsulfat.

In einer Variante des Verfahrens weist das Phenol mindestens eine -O-Alkyl-Gruppe auf.

In einer Variante des Verfahrens ist die Reaktionslösung frei von Übergangsmetallen.

In einer Variante des Verfahrens ist die Reaktionslösung frei von Substraten mit Abgangsfunktionalitäten ungleich Wasserstoffatomen.

In einer Variante des Verfahrens ist die Reaktionslösung frei von organischen Oxidationsmitteln.

In einer Variante des Verfahrens ist das Phenol ausgewählt aus: **Ia**, **IIa, IIIa:**

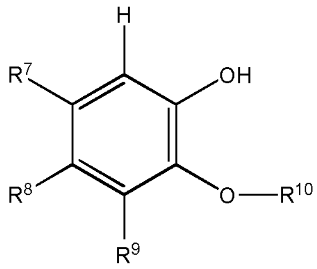

(**Ia**)

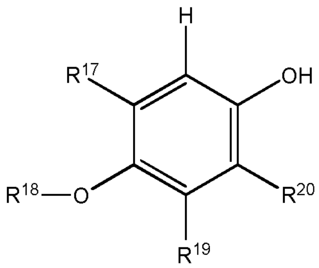

(**IIa**)

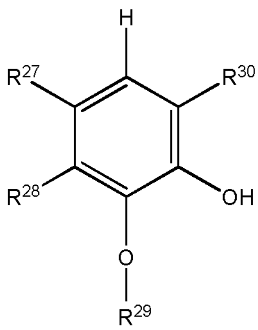

(**IIIa**)

und ist das Naphthol ausgewählt aus: **Ib, IIb, IIIb:**

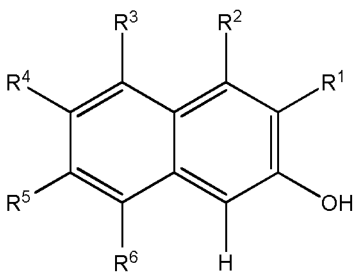

(**Ib**)

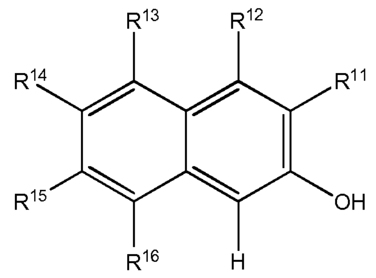

(**IIb**)

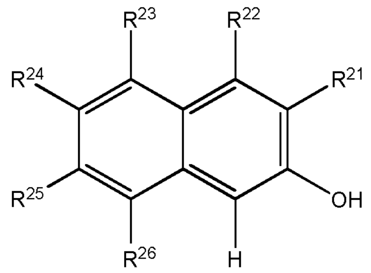

(**IIIb**)

wobei $R^1$, $R^2$, $R^3$, $R^4$, $R^5$, $R^6$, $R^7$, $R^8$, $R^9$, $R^{11}$, $R^{12}$, $R^{13}$, $R^{14}$ $R^{15}$, $R^{16}$, $R^{17}$, $R^{19}$, $R^{20}$, $R^{21}$, $R^{22}$, $R^{23}$, $R^{24}$, $R^{25}$, $R^{26}$, $R^{27}$, $R^{28}$, $R^{30}$ ausgewählt sind aus: -H, -Alkyl, -O-Alkyl, O-Aryl, -S-Alkyl, -S-Aryl und wobei $R^{10}$, $R^{18}$, $R^{29}$ für -Alkyl stehen, und hierbei folgende Kombinationen möglich sind:

| | | | |
|---|---|---|---|
| Phenol | **Ia** | **IIa** | **IIIa** |
| Naphthol | **Ib** | **IIb** | **IIIb** |

Im Folgenden wird die Erfindung anhand von Ausführungsbeispielen und einer Figur näher erläutert.

Die Figur 1 zeigt eine Reaktionsapparatur, in welcher die oben beschriebene Kupplungsreaktion durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

Die Figur 2 zeigt eine Reaktionsapparatur, in welcher die oben beschriebene Kupplungsreaktion im größeren Maßstab durchgeführt werden kann. Die Apparatur umfasst zwei Glasflansche (5'), über die durch Schraubzwingen (2') und Dichtungen Elektroden (3') aus Bor-dotiertem Diamant (BDD) beschichtete Trägermaterialien oder andere, dem Fachmann bekannte, Elektrodenmaterialien angepresst werden. Der Reaktionsraum kann über eine Glashülse (1') mit einem Rückflusskühler versehen werden. Das Reaktionsgemisch wird mit Hilfe eines Magnetrührstäbchens (4') durchmischt.

Die Figur 3 zeigt einen Plot der jeweiligen Oxidationspotentiale unter MeOH-Zugabe (erfolgreiches Beispiel der Kreuzkupplung)

**Analytik**

Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma *Macherey-Nagel GmbH & Co,* Düren durchgeführt. Die Trennungen ohne Druckbeaufschlagung wurden an Kieselgel Geduran Si 60 (0.063-0.200 mm) der Firma *Merck KGaA,* Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.
Zur Dünnschichtchromatographie (DC) wurden PSC-Fertigplatten Kieselgel 60 F254 der Firma *Merck KGaA,* Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf

200 mL Wasser.

Gaschromatographie (GC/GCMS)

Die gaschromatographischen Untersuchungen (GC) von Produktgemischen und Reinsubstanzen erfolgte mit Hilfe des Gaschromatographen GC-2010 der Firma *Shimadzu,* Japan. Es wird an einer Quarzkapillarsäule HP-5 der Firma *Agilent Technologies,* USA (Länge: 30 m; Innendurchmesser: 0.25 mm; Filmdicke der kovalent gebundenen stationären Phase: 0.25 μm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma *Shimadzu,* Japan aufgenommen. Es wird an einer Quarzkapillarsäule HP-1 der Firma *Agilent Technologies,* USA (Länge: 30 m; Innendurchmesser: 0.25 mm; Filmdicke der kovalent gebundenen stationären Phase: 0.25 μm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

Schmelzpunkte

Schmelzpunkte wurden mit Hilfe des Schmelzpunktbestimmungsgerätes SG 2000 der Firma *HW5,* Mainz gemessen und sind unkorrigiert.

Elementaranalyse

Die Elementaranalysen wurden in der analytischen Abteilung des Institutes für Organische Chemie der Johannes Gutenberg-Universität Mainz an einem Vario EL Cube der Firma *Foss-Heraeus,* Haunau angefertigt.

Massenspektrometrie

Alle Elektrosprayionisation-Messungen (ESI+) wurden an einem QTof Ultima 3 der Firma *Waters Micromasses,* Milford, Massachusetts durchgeführt. EI-Massenspektren sowie die hochaufgelösten EI-Spektren wurden an einem Gerät des Typs MAT 95 XL Sektorfeldgerät der Firma *ThermoFinnigan,* Bremen, gemessen.

NMR-Spektroskopie

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde $CDCl_3$ verwendet. Die $^1$H- und $^{13}$C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der $^1$H- und $^{13}$C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

**Allgemeine Arbeitsvorschrift**

Die Kupplungsreaktion wurde in einer Apparatur durchgeführt, wie sie in Figur 1 dargestellt ist. 5 mmol der Verbindung mit dem höheren Oxidationspotential werden mit 15 mmol der Verbindung mit dem niedrigeren Oxidationspotential in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, $10^{-3}$ mbar).

Elektrodenmaterial

| | |
|---|---|
| Anode: | BDD auf Si |
| Kathode: | Ni-Netz |

Elektrolysebedingungen:

| | |
|---|---|
| Temperatur [T]: | 50 °C |
| Stromstärke [I]: | 15 mA |
| Stromdichte [j]: | 2.8 $mA/cm^2$ |
| Ladungsmenge [Q]: | 2 F/mol Unterschusskomponente |
| Klemmspannung [$U_{max}$]: | 3-5 V |

**Synthesen**

1-(2-Hydroxy-3-methoxy-5-methylphenyl)-2-naphthol und 1-(5-Hydroxy-4-methoxy-2-methylphenyl)-2-naphthol

Die Durchführung der Elektrolyse erfolgt gemäß AAV1 in einer ungeteilten Flanschzelle mit BDD- Anode. Hierzu werden 0.78 g (5 mmol, 1.0 Äquiv.) 2-Naphthol und 2.18 g (15 mmol, 3.0 Äquiv.) 4-Methylguajacol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (CH:EE) aufgereinigt und ein Produktgemisch erhalten. Eine zweite "'Flashchromatographie" in Dichlormethan ermöglicht eine Trennung beider Komponenten als leichtrotes kristallines Haupt- und farbloses kristallines Nebenprodukt.

1-(2-Hydroxy-3-methoxy-5-methylphenyl)-2-naphthoi (Hauptprodukt)

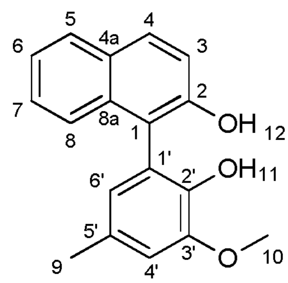

Ausbeute: 899 mg (61%, 3.2 mmol)
GC (Methode *hart,* HP-5): $t_R$= 15.77 min
$R_f$(CH:EE= 4:1)= 0.36, $R_f$(DCM)= 0.36
$m_p$= 145.5 °C (aus DCM/CH umkristallisiert)
$^1$H-NMR (400 MHz, $CDCl_3$) $\delta$= 2.39 (s, 3H, 9-H), 3.96 (s, 3H, 10-H), 5.47-5.52 (m, 1H, 12-H), 5.65- 5.69 (m, 1H, 11-H), 6.75 (d, 1H, 6'-H), 6.85 (d, 1H, 4'-H), 7.32 (dd, 1H, 3-H), 7.34-7.43 (m, 2H, 6-H/7-H), 7.51 (d, 1H, 8-H), 7.83 (s, 1H, 5-H), 7.85 (d, 1H, 4-H);
Kopplungen: $^3J_{3-H, 4-H}$= 9.0 Hz, $^3J_{7-H, 8-H}$= 8.3 Hz, $^4J_{4'-H, 6-H}$= 1.8 Hz;
$^{13}$C-NMR (101 MHz, $CDCl_3$) $\delta$= 21.22 (C-9), 56.08 (C-10), 112.06 (C-4'), 116.62 (C-1), 117.81 (C-3), 119.33 (C-1'), 123.36 (C-6/C-7), 124.42 (C-6'), 124.86 (C-8), 126.48 (C-6/C-7), 128.15 (C-4), 129.18 (C-4a), 129.83 (C-5), 130.36 (C-5'), 133.16 (C-8a), 141.72 (C-2'), 147.24 (C-3'), 150.84 (C-2).
HRMS für $C_{18}H_{16}O_3$ (ESI+) [$M+Na^+$]: ber: 303.0997, gef.: 303.1003
MS (EI, GCMS): m/z(%): 280 (100) $[M]^{+\cdot}$, 265 (12) $[M-CH_3^{\cdot}]^{+\prime}$,249 (12) $[M-OCH_3^{\cdot}]^+$. Elementaranalyse für $C_{18}H_{16}O_3$:

ber: C: 77.12%, H: 5.75%, gef.: C: 76.96%, H: 5.82%.

1-(5-Hydroxy-4-methoxy-2-methylphenyl)-2-naphthol (Nebenprodukt)

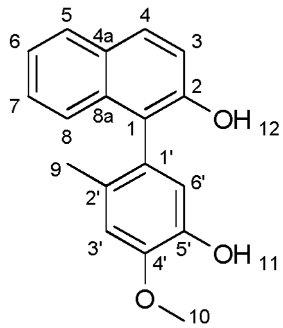

Ausbeute: 106 mg (7%, 0.4 mmol)
GC (Methode *hart,* HP-5): $t_R$= 15.42 min
$R_f$(CH:EE= 4:1)= 0.36, $R_f$(DCM)= 0.32
$m_p$= 182°C (aus DCM/CH umkristallisiert)
$^1$H-NMR (400 MHz, DMSO) $\delta$= 1.82 (s, 3H, H-9), 3.82 (s, 3H, H-10), 6.52 (s, 1H, H-6'), 6.89 (s, 1H, H-3'), 7.14 (dd, 1H, H-8), 7.31-7.18 (m, 3H, H-3/H-6/H-7), 7.75 (d, 1H, H-5), 7.78 (d, 1H, H-4), 8.78 (s, 1H, H-11), 9.31 (s, 1H, H-12); Kopplungen: $^3J_{3-H,\ 4-H}$= 7.1 Hz, $^3J_{5-H,\ 6H}$= 8.7 Hz, $^3J_{7-H,\ 8-H}$= 8.1 Hz, $^4J_{6-H,\ 8-H}$= 1.5Hz;
$^{13}$C-NMR (101 MHz, DMSO) $\delta$= 19.40 (C-9), 56.06 (C-10), 114.31 (C-3'), 118.51 (C-6'), 118.78 (C-3), 120.82 (C-1), 122.78 (C-6), 124.52 (C-8), 126.45 (C-7), 127.84 (C-1'), 128.29, 128.36, 128.70 (C-5/5a/8a), 134.11 (C-2'), 144.52 (C-5'), 147.06 (C-4'), 152.19 (C-2).
HRMS für $C_{18}H_{16}O_3$ (ESI+) [M+Na$^+$]: ber: 303.0997, gef.: 303.1004
MS (EI, GCMS): m/z(%): 280 (100) $[M]^{+\cdot}$, 265 (16) $[M-CH_3^{\cdot}]^{+\cdot}$,249 (12) $[M-OCH_3]^+$. Elementaranalyse für $C_{18}H_{16}O_3$: ber: C: 77.12%, H: 5.75%, gef.: C: 77.19%, H: 5.81%.

1-(3-(Dimethylethyl)-2-hydroxy-5-methoxyphenyl)-2-naphthol

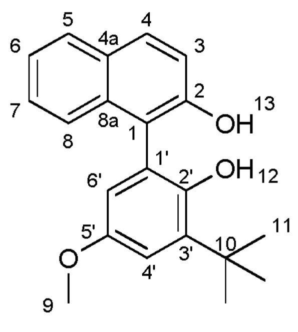

Die Durchführung der Elektrolyse erfolgt gemäß AAV1 in einer ungeteilten Flanschzelle mit BDD-Anode. Hierzu werden 0.72 g (5 mmol, 1.0 Äquiv.) 2-Naphthol und 2.77 g (15 mmol, 3.0 Äquiv.) 2-(Dimethylethyl)-4-methoxyphenol in 27 mL HFIP und 6 mL MeOH gelöst, 0.68 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (CH:EE) aufgereinigt und das Produkt als farbloser Feststoff erhalten.

Ausbeute: 1.05 g (63%, 3.2 mmol)
GC (Methode *hart,* HP-5): $t_R$= 15.75 min
$R_f$(CH:EE= 4:1)= 0.43
$m_p$= 139.9 °C (aus DCM/CH umkristallisiert)

$^{1}$H-NMR (400 MHz, $CDCl_3$) δ= 1.46 (s, 9H, 11-H), 3.77 (s, 3H, 9-H), 4.72 (s, 1H, 2'-H), 5.36 (s, 1H, 2-H), 6.63 (d, 1H, 6'-H), 7.08 (d, 1H, 4'-H), 7.32 (d 1H, 3-H), 7.50-7.35 (m, 3H, 6-H/7-H/8-H), 7.87-7.83 (m, 1H, 5-H), 7.89 (d, 1H, 4-H);
Kopplungen: $^{3}J_{3\text{-H, }4\text{-H}}$= 8.9 Hz; $^{4}J_{4'\text{-H, }6'\text{-H}}$= 3.1 Hz;
$^{13}$C-NMR (101 MHz, $CDCl_3$) δ= 29.41 (C-11), 35.19 (C-10), 55.68 (C-9), 111.95 (C-6'), 114.18 (C-1), 115.87 (C-4'), 117.63 (C-3), 119.16 (C-1'), 123.89, 124.15 (C-6/C-8), 127.38 (C-7), 128.31 (C-5), 129.19 (C-4a), 130.97 (C-4), 132.99 (C-8a), 139.05 (C-3'), 146.93 (C-2'), 151.94 (C-2), 153.41 (C-5').
HRMS für $C_{21}H_{22}O_3$ (ESI+) [$M+Na^+$]: ber: 345.1467, gef.: 345.1465
MS (EI, GCMS): m/z(%): 322 (100) $[M]^{+\cdot}$, 307 (38) $[M-CH_3^{\cdot}]^+$.
Elementaranalyse für $C_{21}H_{22}O_3$: ber: 78.23%, H: 6.88%, gef.: C: 78.18%, H: 6.82%.

**Ergebnisse**

In Tabelle 1 sind die Ausbeuten und Selektivitäten aufgelistet:

Tabelle 1:

| Produkt | Lösungsmittel | Ausbeute (isoliert)[a] | Selektivität (AB:BB)[b] |
|---|---|---|---|
| 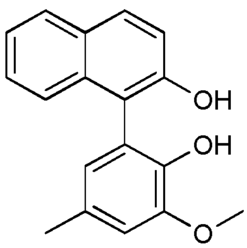 | HFIP + 18% MeOH | 61% | >100:1 |
| | HCOOH + 9% MeOH | 45% | |
| 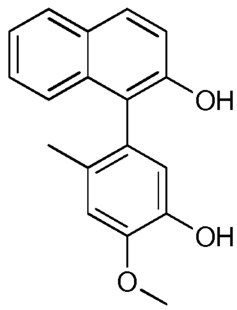 | | 7% | |
| 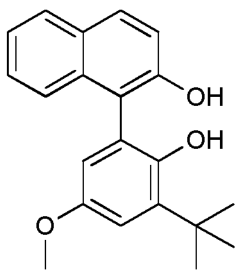 | HFIP + 18% MeOH | 63% | >100:1 |
| | HCOOH + 9% MeOH | 34% | >100:1 |
| Elektrolyseparameter: n(Phenol1)= 5 mmol, n(Phenol2)= 15 mmol, Leitsalz: MTES, c(MTES)= 0.09 M, V(Lösungsmittel)= 33 mL, Anode: BDD/Si, Kathode: Ni-Netz, j= 2.8 $mA/cm^2$, T= 50 °C, Q= 2 F*n(Phenol1). Die Elektrolyse erfolgt galvanostatisch.<br>[a]: isolierte Ausbeute bezogen auf n(Phenol1);<br>[b]: via GC bestimmt.<br>AB: Kreuzkupplungsprodukt,<br>BB: Homokupplungsprodukt. | | | |

Tabelle 1 zeigt, dass verschiedene Phenole mit unterschiedlichen Naphtholen mit der oben beschriebenen Methode zur direkten Kreuzkupplung gebracht werden können.

### Einfluss der Oxidationspotentialdifferenzen auf Ausbeuten und Selektivitäten

Cyclovoltammetrische Messungen eingesetzter Substrate ergeben, dass Unterschiede einzelner Oxidationspotentialdifferenzen (im weiteren $\Delta E_{Ox}$ genannt) mit Selektivitäten und Ausbeuten der elektrochemischen Kreuzkupplung von Phenolen korrelieren.

Tabelle 2:

| Kupplungspartner | **HFIP pur** | **18% MeOH** | **HFIP pur** | **18% MeOH** |
|---|---|---|---|---|
| $\Delta E_{Ox}$ mit 2-Naphthol | 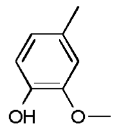 | | 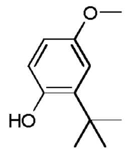 | |
| | GC: 20% + 5% NP<br>Δ = -0.02 | GC: 21%<br>Δ = 0.05 | GC: 25% + 4% NP<br>Δ = 0.05 V | GC: 25%<br>Δ =0.13 V |
| | | | | |
| Kupplungspartner | **HFIP pur** | **18% MeOH** | **HFIP pur** | **18% MeOH** |
| $\Delta E_{Ox}$ mit 2-Naphthol | 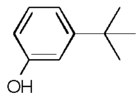 | | 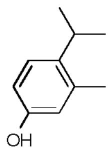 | |
| | GC: 0%<br>Δ = -0.27 V | GC: 0%<br>Δ = -0.20 V | GC: 0%<br>Δ = -0.27 V | GC: 0%<br>Δ = -0.17 V |

Arbeitselektrode: Glaskohlenstoff, Gegenelektrode: Glaskohlenstoff, Referenzelektrode: Ag/AgCl in ges. LiCl/EtOH, v = 10 mV/s, Oxidationskriterium: j = 0.10 mA/cm$^2$, c(Phenol) = 0.152 M, Leitsalz: MTES, c(MTES) = 0.09M. Lösungsmittel: HFIP. $\Delta E_{Ox}$ = Ox.Pot.$_{Kupplungspartner}$-Ox.Pot.$_{Tabelleneintrag}$. NP: Nebenprodukt; Gaschromatographische Integration von Produktverhältnissen.

Durch Zugabe von MeOH (Eintrag 1) wird eine Umkehr der Selektivität für die Phenoloxidation erreicht. Hierdurch ist eine Unterbindung der Naphthol-Homokupplung möglich. Durch Vergrößerung von $\Delta E_{Ox}$ (Eintrag 2, HFIP/MeOH-System) wird eine selektive Bildung des Kreuzkupplungsproduktes möglich.

Durch den Zusatz von Methanol laufen die beiden Oxidationspotentiale weit genug auseinander (siehe Figur 3), so dass erfolgreich die sehr selektive Kreuzkupplung elektrochemisch durchgeführt werden kann.

## Patentansprüche

1. Elektrochemisches Verfahren umfassend die Verfahrensschritte:

    a) Einfüllen eines Lösungsmittels oder Lösungsmittelgemisches sowie eines Leitsalzes, in ein Reaktionsgefäß,
    b) Zugabe eines Phenols mit einem Oxidationspotential $|E_{Ox}1|$ in das Reaktionsgefäß,
    c) Zugabe eines Naphthols mit einem Oxidationspotential $|E_{Ox}2|$ in das Reaktionsgefäß, wobei die Substanz mit dem höheren Oxidationspotential im Überschuss zugesetzt wird,
    und es gilt: $|E_{Ox}1| - |E_{Ox}2| = |\Delta E|$
    und das Lösungsmittel oder Lösungsmittelgemisch so gewählt ist, dass $|\Delta E|$ im Bereich von 10 bis 450 mV liegt,
    d) Einbringen zweier Elektroden in die Reaktionslösung,
    e) Anlegen einer Spannung an die Elektroden,
    f) Kupplung des Phenols mit dem Naphthol zu einem kreuzgekuppeltem Produkt.

2. Verfahren nach Anspruch 1,
    wobei die Substanz mit dem höheren Oxidationspotential mindestens in der doppelten Menge gegenüber der Substanz mit dem niedrigeren Oxidationspotential eingesetzt wird.

3. Verfahren nach einem der Ansprüche 1 oder 2, wobei $|\Delta E|$ in einem Bereich von 20 mV bis 400 mV liegt.

4. Verfahren nach einem der Ansprüche 1 bis 3, wobei das Phenol mindestens eine -O-Alkyl-Gruppe aufweist.

5. Verfahren nach einem der Ansprüche 1 bis 4, wobei die Reaktionslösung frei von Übergangsmetallen ist.

6. Verfahren nach einem der Ansprüche 1 bis 5, wobei die Reaktionslösung frei von organischen Oxidationsmitteln ist.

7. Verfahren nach einem der Ansprüche 1 bis 6, wobei das Phenol ausgewählt ist aus: **Ia**, **IIa, IIIa:**

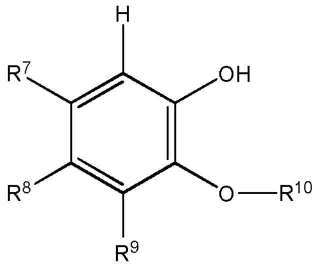

(**Ia**)

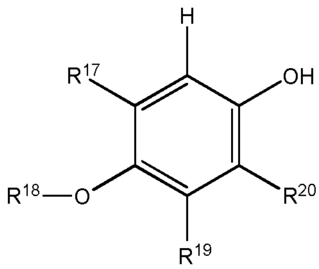

(**IIa**)

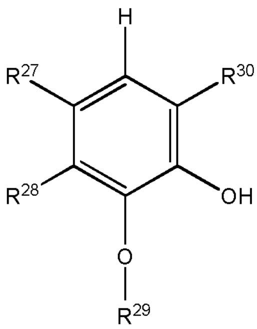

(**IIIa**)

und wobei das Naphthol ausgewählt ist aus: **Ib, IIb, IIIb:**

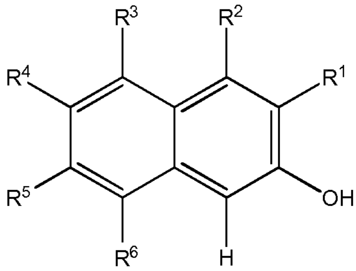

(**Ib**)

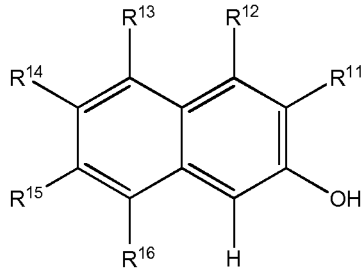

(**IIb**)

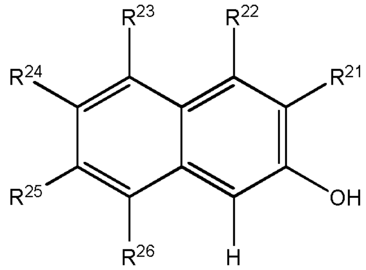

(**IIIb**)

wobei $R^1$, $R^2$, $R^3$, $R^4$, $R^5$, $R^6$, $R^7$, $R^8$, $R^9$, $R^{11}$, $R^{12}$, $R^{13}$, $R^{14}$ $R^{15}$, $R^{16}$, $R^{17}$, $R^{19}$, $R^{20}$, $R^{21}$, $R^{22}$, $R^{23}$, $R^{24}$, $R^{25}$, $R^{26}$, $R^{27}$, $R^{28}$, $R^{30}$ ausgewählt sind aus: -H, -Alkyl, -O-Alkyl, -S-Alky, -S-Aryl- und wobei $R^{10}$, $R^{18}$, $R^{29}$ für-Alkyl stehen, und hierbei folgende Kombinationen möglich sind:

| | | | |
|---|---|---|---|
| Phenol | **Ia** | **IIa** | **IIIa** |
| Naphthol | **Ib** | **IIb** | **IIIb** |

## Claims

1. Electrochemical process comprising the process steps of:

   a) introducing a solvent or solvent mixture and a conductive salt into a reaction vessel,
   b) adding a phenol having an oxidation potential $|E_{Ox}1|$ to the reaction vessel,
   c) adding a naphthol having an oxidation potential $|E_{Ox}2|$ to the reaction vessel, the substance having the higher oxidation potential being added in excess,
   and where: $|E_{Ox}1| - |E_{Ox}2| = |\Delta E|$
   and the solvent or solvent mixture is selected such that $|\Delta E|$ is in the range from 10 to 450 mV,
   d) introducing two electrodes into the reaction solution,
   e) applying a voltage to the electrodes,
   f) coupling the phenol to the naphthol to give a crosscoupled product.

2. Process according to Claim 1,
   wherein the substance having the higher oxidation potential is used at least in twice the amount relative to the substance having the lower oxidation potential.

3. Process according to either of Claims 1 and 2,
   wherein $|\Delta E|$ is within a range from 20 mV to 400 mV.

4. Process according any of Claims 1 to 3,
   wherein the phenol has at least one -O-alkyl group.

5. Process according any of Claims 1 to 4,
   wherein the reaction solution is free of transition metals.

6. Process according any of Claims 1 to 5,

wherein the reaction solution is free of organic oxidizing agents.

7. Process according any of Claims 1 to 6, wherein the phenol is selected from: **Ia, IIa, IIIa:**

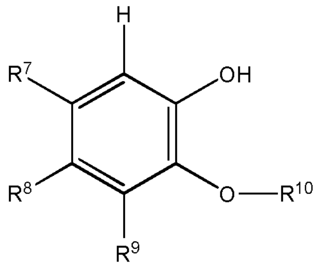

(**Ia**)

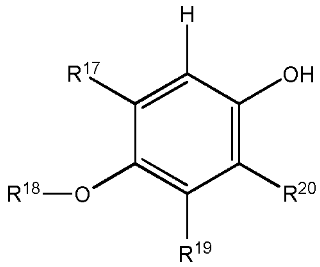

(**IIa**)

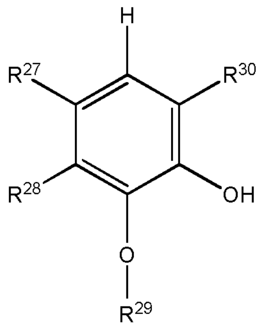

(**IIIa**)

and wherein the naphthol is selected from: **Ib, IIb, IIIb:**

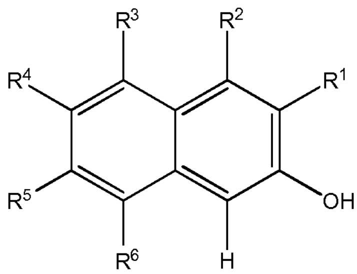

(**Ib**)

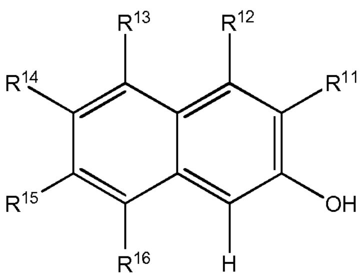

(**IIb**)

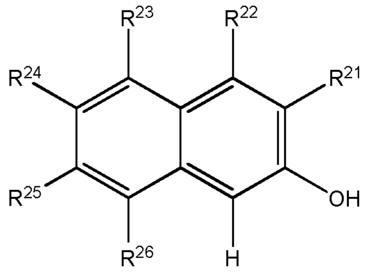

(**IIIb**)

where $R^1$, $R^2$, $R^3$, $R^4$, $R^5$, $R^6$ $R^7$, $R^8$, $R^9$, $R^{11}$, $R^{12}$, $R^{13}$, $R^{14}$, $R^{15}$, $R^{16}$, $R^{17}$, $R^{19}$, $R^{20}$, $R^{21}$, $R^{22}$, $R^{23}$, $R^{24}$, $R^{25}$, $R^{26}$, $R^{27}$, $R^{28}$, $R^{30}$ are selected from: -H, -alkyl, -O-alkyl, -S-alkyl, - S-aryl- and
where $R^{10}$, $R^{18}$, $R^{29}$ are -alkyl,
and the following combinations are possible here:

| | | | |
|---|---|---|---|
| phenol | **Ia** | **IIa** | **IIIa** |
| naphthol | **Ib** | **IIb** | **IIIb** |

**Revendications**

**1.** Procédé électrochimique comprenant les étapes de procédé suivantes :

a) le remplissage d'un solvant ou d'un mélange de solvants, ainsi que d'un sel conducteur, dans un récipient de réaction,
b) l'ajout d'un phénol ayant un potentiel d'oxydation $|E_{ox}1|$ dans le récipient de réaction,
c) l'ajout d'un naphtol ayant un potentiel d'oxydation $|E_{ox}2|$ dans le récipient de réaction, la substance ayant le potentiel d'oxydation le plus élevé étant ajoutée en excès, avec : $|E_{ox}1|-|E_{ox}2|=|\Delta E|$,
et le solvant ou le mélange de solvants étant choisi de sorte que $|\Delta E|$ se situe dans la plage allant de 10 à 450 mV,
d) l'introduction de deux électrodes dans la solution de réaction,
e) l'application d'une tension aux électrodes,
f) le couplage du phénol avec le naphtol pour former un produit couplé croisé.

**2.** Procédé selon la revendication 1, dans lequel la substance ayant le potentiel d'oxydation le plus élevé est utilisée au moins en une quantité double de la substance ayant le potentiel d'oxydation le plus faible.

**3.** Procédé selon l'une quelconque des revendications 1 ou 2, dans lequel $|\Delta E|$ se situe dans une plage allant de 20 mV à 400 mV.

**4.** Procédé selon l'une quelconque des revendications 1 à 3, dans lequel le phénol comprend au moins un groupe -0-alkyle.

**5.** Procédé selon l'une quelconque des revendications 1 à 4, dans lequel la solution de réaction est exempte de métaux de transition.

**6.** Procédé selon l'une quelconque des revendications 1 à 5, dans lequel la solution de réaction est exempte d'oxydants organiques.

**7.** Procédé selon l'une quelconque des revendications 1 à 6, dans lequel le phénol est choisi parmi : Ia, IIa, IIIa :

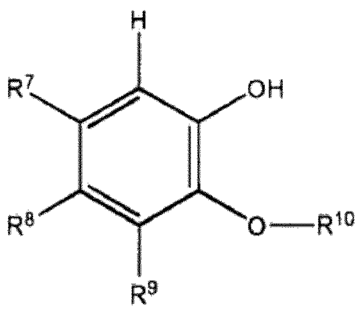

(Ia)

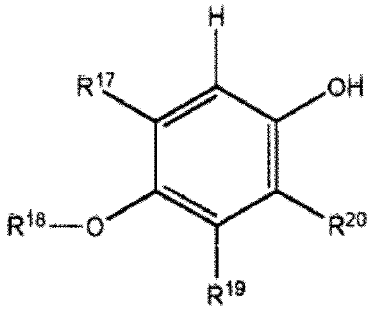

(IIa)

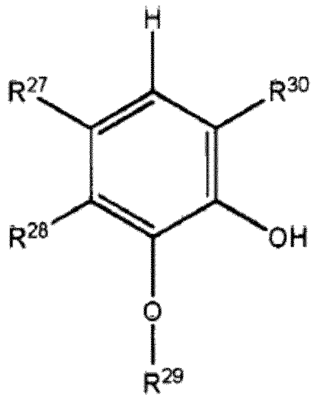

(IIIa)

et dans lequel le naphtol est choisi parmi : Ib, IIb, IIIb :

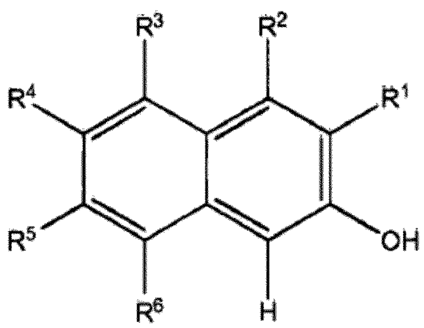

(Ib)

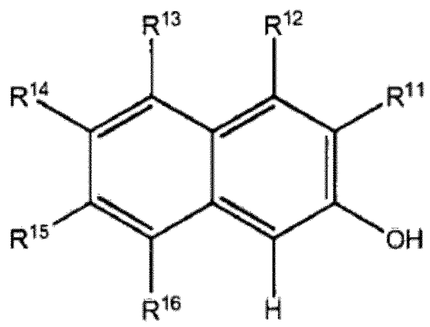

(IIb)

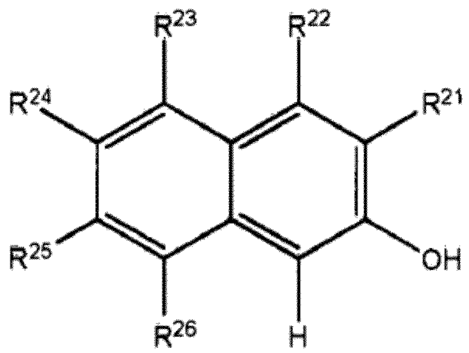

(IIIb)

$R^1$, $R^2$, $R^3$, $R^4$, $R^5$, $R^6$ $R^7$, $R^8$, $R^9$, $R^{11}$, $R^{12}$, $R^{13}$, $R^{14}$, $R^{15}$, $R^{16}$, $R^{17}$, $R^{19}$, $R^{20}$, $R^{21}$, $R^{22}$, $R^{23}$, $R^{24}$, $R^{25}$, $R^{26}$, $R^{27}$, $R^{28}$, $R^{30}$ étant choisis parmi : -H, -alkyle, -O-alkyle, -S-alkyle, -S-aryle, et $R^{10}$, $R^{18}$, $R^{29}$ représentant -alkyle, et les combinaisons suivantes sont possible :

| | | | |
|---|---|---|---|
| phénol | Ia | IIa | IIIa |
| naphtol | Ib | IIb | IIIb |

Figur 1

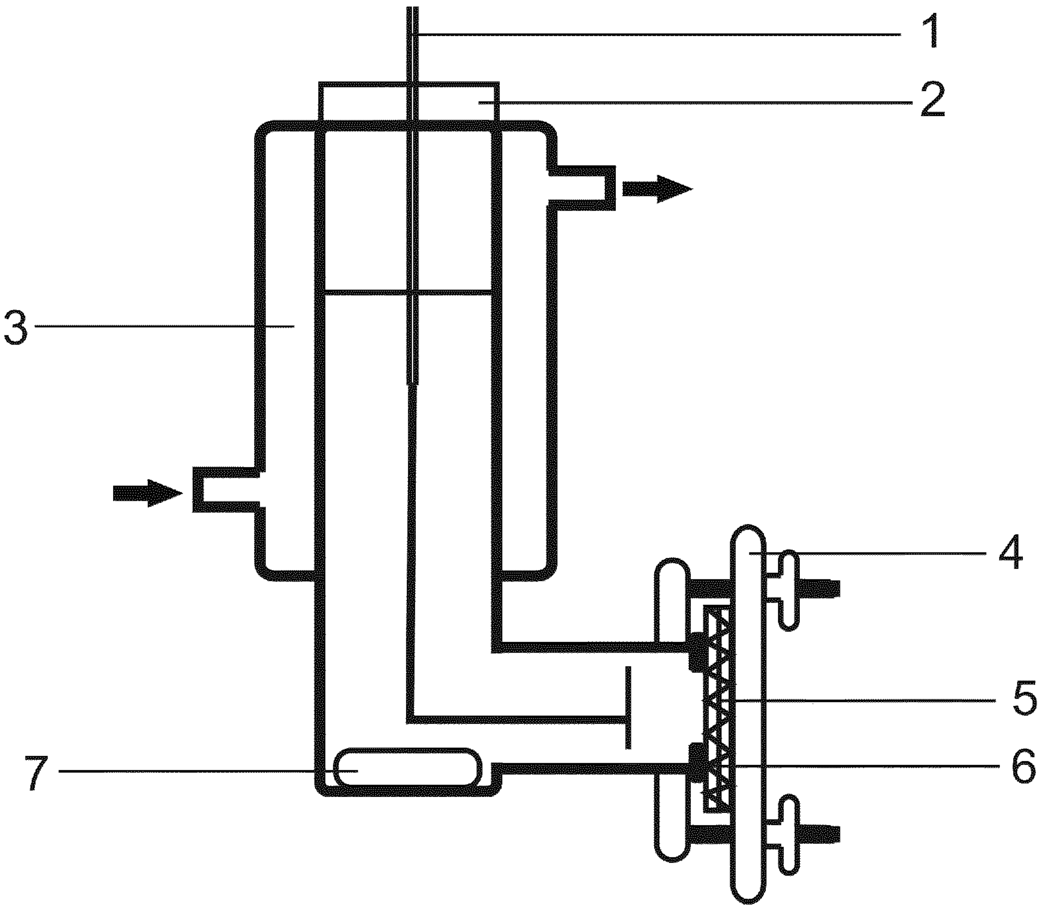

Figur 2

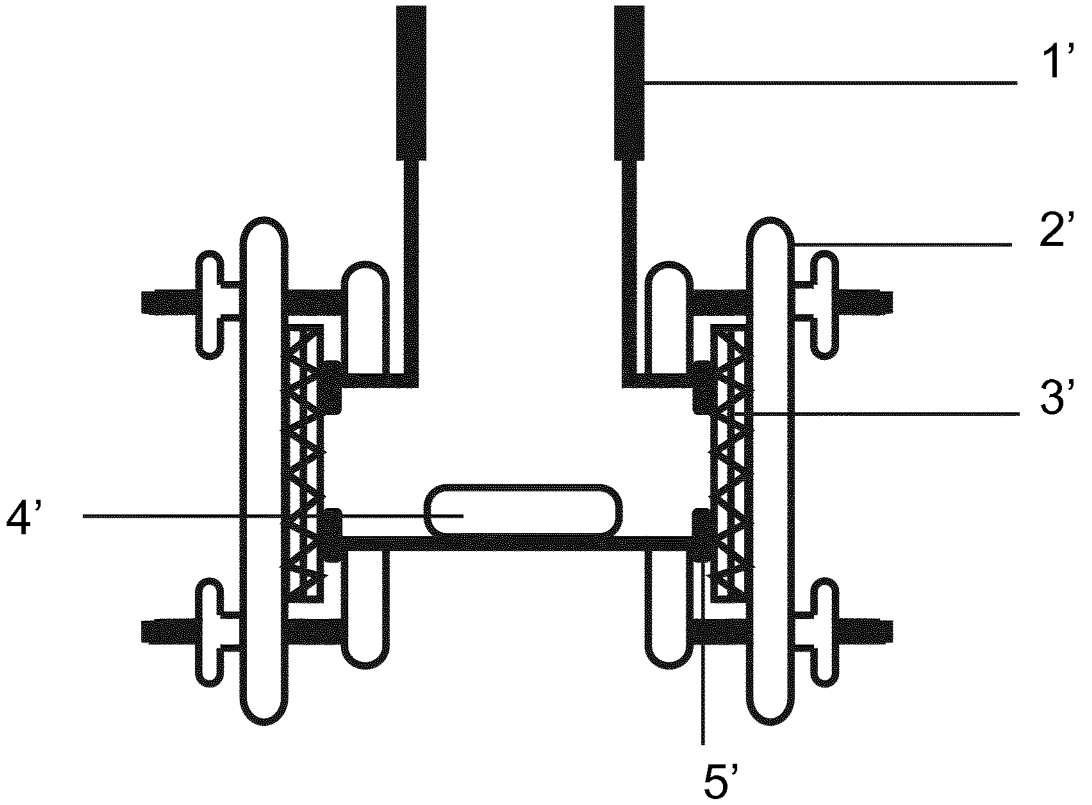

Figur 3

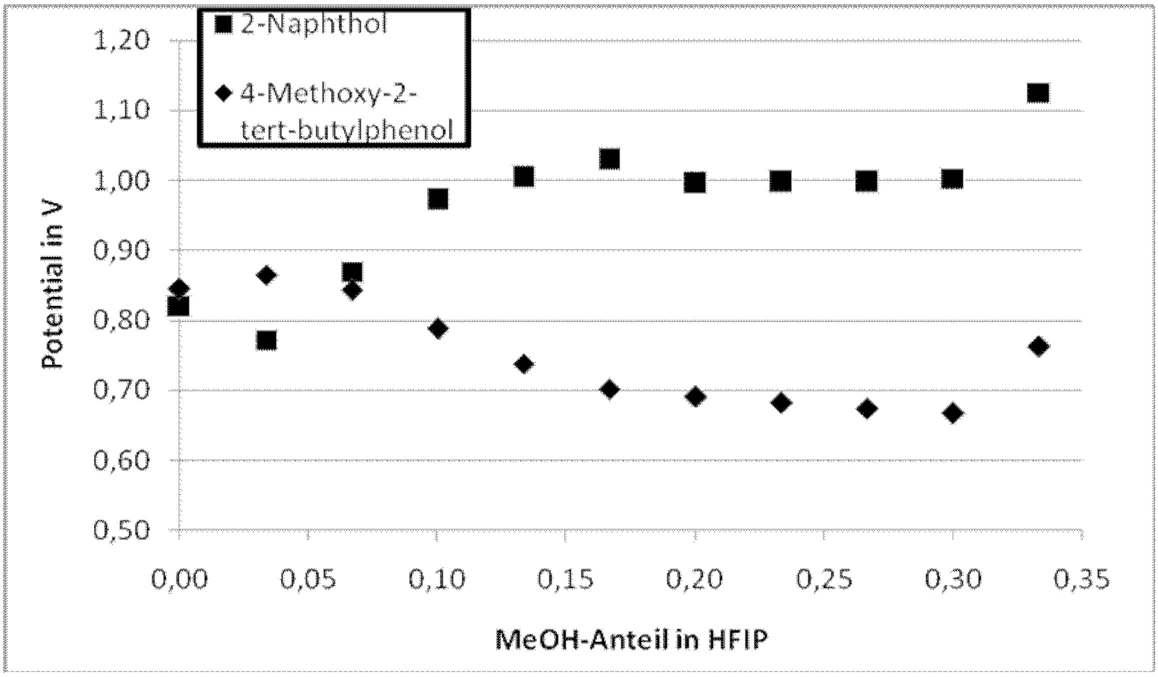
2-Naphthol
4-Methoxy-2-tert-butylphenol
1,20
1,10
1,00
0,90
0,80
0,70
0,60
0,50
Potential in V
0,00
0,05
0,10
0,15
0,20
0,25
0,30
0,35
MeOH-Anteil in HFIP

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010139685 A1


### In der Beschreibung aufgeführte Nicht-Patentliteratur


- **KIRSTE et al.** *Angew. Chem.,* 2010, vol. 122, 983-987
- **KIRSTE et al.** *J. Am. Chem. Soc.,* 2012, vol. 134, 3571-3576
- Anodic Phenol-Arene Cross-Coupling Reaction on Boron-Doped Diamond Electrodes. **KIRSTE A et al.** ANGEWANDTE CHEMIE. INTERNATIONAL EDITION. VCH VERLAG, vol. 49, 971-975